# EUROPEAN PATENT SPECIFICATION

(11) **EP 0 613 375 B1**
(45) Date of publication and mention of the grant of the patent: **03.03.2004**
(21) Application number: 92923065.4
(22) Date of filing: 28.10.1992
(51) Int. Cl.: A61K 38/00, A61K 38/02, C07K 5/00, C07K 7/00, C07K 17/00, C07K 7/06, A61P 37/00

(54) **PHARMACEUTICAL LYSINE-CONTAINING POLYPEPTIDE COMPOSITIONS AND THE USE THEREOF**
PHARMAZEUTISCHE LYSIN ENTHALTENDE POLYPEPTID-ZUSAMMENSETZUNGEN UND VERFAHREN ZU DEREN VERWENDUNG
COMPOSITIONS POLYPETIDIQUES PHARMACEUTIQUES CONTENANT DE LA LYSINE ET PROCEDES D'UTILISATION DESDITES COMPOSITIONS

(30) Priority: 28.10.1991 US 783517
(43) Date of publication of application: 07.09.1994
(73) Proprietor: CYTRAN LTD., Hamilton HM11 (BM)
(72) Inventor: GREEN, Lawrence, R., Tacoma, WA 98498 (US); IVANOV, Vadim T., Moscow 117296 (SU); MIKHALYOVA, Inessa I., Moscow 117437 (SU); VASKOVSKY, Boris V., Moscow 113162 (SU); MIKHALTSOV, Alexander N., St. Petersburg 190031 (SU); KHAVINSON, Vladimir K., St. Petersburg 197198 (SU); MOROZOV, Vyacheslav G., St. Petersburg 197198 (SU)
(74) Representative: Beresford, Keith Denis Lewis
(86) International application number: PCT/US1992/009252
(87) International publication number: WO 1993/008816

(56) References cited:
- EP-A- 0 164 654
- GB-A- 1 363 801
- US-A- 4 395 404
- MECHANISMS OF AGEING AND DEVELOPMENT, vol. 49, no. 3, September 1989 pages 245-257, V.N. ANISIMOV ET AL. 'Effect of Low-Molecular Weight Factors of Thymus and Pineal Gland on Life Span and Spontaneous Tumour Development in Female Mice of Different Age'
- CHEMICAL ABSTRACTS, Vol. 111, No. 7, issued 14 August 1989, R.S. CHEN, "Monte Carlo simulations for the study of hemoglobin-fragment conformations", see page 541, column 2, Abstract No. 55230R, J. Comput. Chem., 10(4), 488-494.
- CHEMICAL ABSTRACTS, Vol. 82, No. 11, issued 17 March 1975, FAUSZT et al., "Synthesis of a proposed porcine growth hormone-releasing hormone (GH-RH) and the N-terminal decapeptide of the beta chain of human hemoglobin", see page 487, column 2, Abstract No. 73453C, Acta Chim. Acad. Sci. Hung., 82(4), 471-480.

## Description

The present invention is directed to peptide pharmaceutical compositions and uses thereof, in particular, peptides including the amino acid sequence, -L-Glx-L-Glx-L-Lys-. These peptide compositions are useful in the treatment of immunodepressed states and of opportunistic infections in immunodepressed states associated with acquired immune deficiency syndrome.

### BACKGROUND OF THE INVENTION

The present invention is based in part on the discovery that certain peptide compositions, in particular pentapeptides, exhibit a broad range of efficacy for the prevention and treatment of opportunistic infections in immunodepressed states, and for therapeutically effective treatment of immunodeficient states, particularly AIDS. This is believed to be highly unexpected for such relatively small compounds to exhibit such a broad range of activity. Furthermore, we have not found any significant side effects from the use of the peptides according to the present invention. Due to their simple nature, the peptides of the present invention are relatively inexpensive to manufacture.

EP0164654 describes the manufacture of medicaments containing peptides for the treatment of viral or fungoidal immune deficiencies, chronical infects, autoimmune diseases and for the therapy of diseases caused by cells which contains immunological relevant changes of membranes.

GB1363801 describes the manufacture of substantially pure peptides which can be administered by injection or by absorption through mucous membranes, useful for inducing hormone release especially growth hormone.

US4,395,404 discloses several peptide factors present in the thymus gland which have been implicated to play important roles in the development and maintenance of immunological competence in man and in animals.

Chemical Abstracts, vol 111, no. 7, abstract no. 55230r discloses a peptide (Thr-Pro-Glu-Glu-Lys) for the stimulation of conformation of haemoglobin-fragments.

Chemical Abstracts, vol. 82. no. 11, abstract no. 73454c discloses the synthesis of a porcine growth hormone-releasing hormone and the N-terminal decapeptide of the β-chain of human haemoglobin.

As used herein, the terms "immunomodulator" and "immunomodulating" encompass the activity of enhancing or restoring the subject's immune system, as evidenced by measurable blood parameters and/or the patient's improved ability to combat infection or disease, and the ability to heal tissue. Hence, immunomodulation encompasses improvement of the immune system due to an immunodeficient state (for example, caused by removal of the thymus), and/or an immunodepressed state (for example, caused by exposure to radiation). Furthermore, the present invention is useful for modulation of the immune system by lowering blood parameters and other indicia of the immune state if these indicia are abnormally elevated. The present invention is useful in a therapeutic method of treating the immunodeficient, immunodepressed or elevated immune state per se, thus providing prophylaxis against infection and disease, as well as in a treatment of infection, disease or wound indirectly by enhancing the immune system.

It is therefore an object of the present invention to provide pharmaceutical compositions of peptides that have broad immunomodulating activity, as well as activity for other uses such as treatment of infections, enhancement of metabolic processes, and many other uses.

This and other objects will be apparent from the following description and appended claims.

### DESCRIPTION OF THE PREFERRED EMBODIMENTS

In a first aspect, the inventions is a substantially pure compound of at least five amino acids of the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

In a second aspect, the invention is a pharmaceutical composition comprising a pharmaceutically acceptable vehicle and an effective amount of a substantially pure compound of the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

In a third aspect, the invention relates to use of a substantially pure compound for the manufacture of a pharmaceutical composition for the treatment of an immunodeficient, immunodepressed or elevated immune state wherein the compound has the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

The peptides according to the present invention may be formulated into any convenient formulation which allows for the active ingredient to be absorbed into the blood stream. Intramuscular and intranasal forms of application are preferred. The preferred dosage rate of the active ingredient for intramuscular administration is about 50 to 100µg per dose for adults (for a 300 to 1000µg total treatment therapy); for infants up to 1 year old about 10µg per dose, for infants 1 to 3 years old about 10 to 20µg per dose; for infants 4 to 6 years old about 20 to 30µg per dose, for children 7 to 14 years old about 50µg per dose. All of the foregoing dosages are useful for a treatment of 3 to 10 days, depending upon the immunodeficiency level. The treatment may be repeated as needed, usually within 1 to 6 months.
For prophylactic uses against opportunistic infections in immunodeficient or immunodepressed patients, the intramuscular and/or intranasal single daily dose for adults may be from about 50 to 100µg, and for children about 10 to 50 µg per dose for treatment over 3 to 5 days.

For treatment of external infections, the peptides may be applied in single daily dosages of about 10µg (over 4 to 10 days) or as installations into the site of infection at about 5µg twice daily over about 4 to 5 days.

The peptides may be utilized intramuscularly as an injection solution with the active ingredient in a therapeutically effective immunopotentiating amount of about .001 to .01% by weight. If prepared in the form of a tablet, capsule or suppository, it is preferred that the active ingredient be present in an amount of about 0.1mg per tablet, suppository or capsule. In such form, the capsule, suppository or tablet may also contain other conventional excipients and vehicles such as fillers, starch, glucose, etc.

The peptides may be obtained by conventional peptide synthesis, including the Merrifield solid state peptide synthesis technique. For example, an amino and side chain protected derivative of an activated ester of Glx is reacted with side-group protected L-Lys, attached to the solid phase at its C-terminus. After elimination of the alpha-amino protecting group, the next amino acid is added, etc. Cleavage of the peptides, removal of protection groups use purification, lyophilization, gel purification, and the like results in the desired product.

The active polypeptide ingredients of the pharmaceutical preparations according to the present invention may be used as free peptides or in the form of a water soluble pharmaceutically acceptable salt, such as a sodium, potassium, ammonium or zinc salt. It will be understood that the peptides may be administered with other active ingredients which independently impart an activity to the composition, such as antibiotics, interferon, anesthetics, and the like.

The most preferred formulation according to the present invention is a solution for intramuscular injection containing about .001 to .01% by weight (.0001-.001mg/kg body weight, or 10-100µg active ingredient per 1ml solvent). The pharmaceutically acceptable vehicle for this injectable form may be any pharmaceutically acceptable solvent such as 0.9% aqueous sodium chloride, distilled water, Novocaine solution, Ringer's solution, glucose solution, and the like. The peptide containing compositions according to the present invention may be administered in a compatible pharmaceutical that is suitable for parenteral administration (e.g., intravenous, subcutaneous, intramuscular). The preparations may be subjected to conventional pharmaceutical operations, such as sterilization, and may contain adjuvants, such as preservatives, stabilizers, wetting agents and the like.

The pharmaceutical preparations according to the present invention demonstrate a high effectiveness in the treatment of immunodepressed and immunodeficient states for the preventing and treatment of opportunistic infections in those states.

Also included within the scope of the present invention are the pharmaceutically acceptable salts of the peptide, such as sodium or potassium or strong organic bases, such as guanidine.

The peptides containing compositions according to the present invention have activity in the restoration and stimulation of the immune functions. Thus they are useful in the treatment of opportunistic infections of an immunodepressed subject in an immunopotentiating effective amount as described above.

The peptide compositions according to the present invention may also be used in veterinary practice as an immunomodulatory agent for prophylaxsis and treatment of hypotrophy in farming animals, fur bearing animals and poultry.

Among the opportunistic infections which may be treated utilizing the compositions according to the present invention are: respiratory diseases, influenza, AIDS, burns, wounds, other open sores, rashes (due to allergic reactions), sun exposure, local trauma (with an ointment), eczemas, psoriasis, and the like. Furthermore, the compositions according to the present invention may be utilized to assist healing in immunodepressed or immunodeficient states, such as for the healing of bone fractures, lesions, gingival diseases, gynecological infections, infralymphatic infections, and the like. The compositions may also be used to enhance the immunodeficient state to increase susceptibility to microbial antibiotics and to enhance the patient's responsive reaction to other types of therapies.

The compostions of the present invention are useful for the treatment and prevention of states and diseases associated with homopoiesis reduction. In general, the compositions are useful for treatment and prevention of immunodepressive and immunodefficient states, primary or caused by acute or chronic diseases, including inflammatory infectitous and other diseases which may be accompanied by toxemia.

The compositions of the present invention are particularly useful for the treatment and prevention of anemias of all types such as those caused by blood loss, blood formation affection or blood destruction enhancement, including all types of iron deficient anemia, anemia associated with heme synthesis disorders, hemolytic and aplastic anemia, and primary and secondary anemia. Iron deficient anemia includes chronic blood by menstruation, gastrointestinal bleeding, hemoptysis, hematuria, hemodialysis, malabsorption, infections, inborn iron deficiency, dietary iron deficiency and increased iron requirements during infancy, adolescence, pregnancy and lactation. Anemia also includes inherited hemolytic anemia such as those associated with abnormalities of the red cell membrane such as spherocytosis, stomatocytosis and elliptocytosis; anemia associated with erythrocytes enzyme deficiencies (glucose-6-phosphate dehydrogenase deficiency, pyruvate kinase deficiency, etc.) and also anemia associated with abnormal structure and synthesis of hemoglobin chains (such as thalassemia, sickle cell diseases, etc.). These hemolytic anemias also are treatable when associated with the symptom of antibody attack against erythrocytes or erythroid bone marrow cells, anemia associated with red cell membrane changes due to somatic mutations, paroxysmalnocturnal hemoglobinuria, anemia associated with mechanical red cell damage (artificial valves), anemia associated with chemical red cell damage (hemolytic poisons, lead, etc.) anemia associated with Vitamin E deficiency, anemia associated with thermal red cell damage and with parasites (including malaria), infections and hypersplenism. Anemia also includes megaloblastic anemia such as those associated with vitamin B12 deficiency, addisonian/pernicious anemia , cancer of the stomach, intestinal diseases (intestinal tumors, sprue, terminal ileitis), gastrointestinal operations (gastroenterostomy, blind loop syndrome, resection of the ileum, etc.). Anemia conditioned by increase vitamin B12 expenditure and effects thereof on the bone marrow, including causes such as intestinal parasites (diphyllobothriasis), and liver diseases (hepatitis, cirrhosis, etc.), and hemoblastosis.

Other anemia may be caused by folic acid deficiency associated with intestinal diseases (intestinal surgery, intestinal malabsorption), long term drug administration (anti convulsants, etc.), dietary insufficiency, increased folic demand (infancy, adolescence, pregnancy, lactation), hemolytic anemia, psoriasis , excess loss of folate (hemodialysis, peritoneal dialysis), and defective folate synthesis (liver disease, alcoholism, antifolate drugs).

The invention is also useful for the treatment of T-cell immunodeficiencies such as severe combined immunodeficiency, Di George's syndrome, Wiskott-Aldrich syndrom, and chronic mucocutaneous candidiasis.

The peptides according to the present invention are also useful for the treatment and prevention of diseases which manifest bone marrow functional insufficiency (including aplastic anemia), such as that associated with radiation, chemical agents (benzene, trinitrotoluene, insecticides, etc.), antibodies against marrow cells, hereditary factors (Fanconi's anemia) or infections such as viral hepatitis.

In general, the peptides according to the present invention are useful for the treatment and prevention of all types of lympho- leuko- and cytopenias, including congenital conditions, and those caused by radiation or other cytotoxic factors and agents.
Other conditions which may be treated include hemorrhagic diathesis, hemophilia, thrombocytopenic purpura, hemorrhagic vasculitis, DC, hereditary hemorrhagic telangiectasia.

Organ and tissue regeneration may also be stimulated by use of the peptides according to the present invention in states and diseases accompanied by restoration of tissue and integrity, including wound processes and toxic and infectious damage of cells. Also, the immune system and other defenses may be stimulated by the peptides according to the present invention during recovery to combat chronic diseases accompanied by toxemia (or tissue metabolism disturbances) or to assist in the adaptation to a new or extreme environmental condition. Immunodeficiencies associated with the use of drugs, including antibiotics and antineoplastics, or associated with the use of therapies, such as radiation and surgical intervention may also be alleviated by use of the peptides according to the present invention.

Tumor diseases and parasitic diseases, (such as helminthism) may also be treated as well as specific infections, such as tuberculosis, syphilis and gout. Acute and chronic poisoning may also be treated.

Agranulocytosis resulting from cytostatic factors (irradiation, cytotoxic drugs, drugs with cytotoxic side effects [such as chloramphenicol, chlorpromazine, etc.]) may also be treated. Immune agranulocytoses may also be treated and prevented, including those in systemic diseases, (SLE, hepatitis, etc.) or those caused by formation of anti-leukocytic antibodies under the influence of chemical compounds, such as drugs (sulfamides, barbiturates, aminopyrine, etc.).

States and diseases accompanied by neutropenia, may also be treated including infectious diseases (typhoid, tularemia, brucellosis), viral diseases (hepatitis, influenza, mumps, infectious mononucleosis), protozoa (malaria), chemical and physical agents (radiation, drugs), idiosyncratic drug reactions, hypersplenism (liver diseases, storage diseases), collagen-vascular diseases (SLE), severe folic acid or B12 deficiency, and neutropenia associated with extracorporalicirculation and pulmonary microcirculation disorders.

Pancytopenia may also be treated caused by hemopoiesis (megaloblastic anemia and myelodysplactic syndromes), hemodilution, hypersplenism and immune destruction.

Secondary immunodeficiencies such as viral infections (HIV, measles, cytomegalovirus, Epstein-Barr virus), splenectomy, burns, frost bites, wound healing processes, immunosuppressive drugs (antibiotics, corticosteroids, antimetabolites, etc.), radiation, prematurity, diabetes, protein-losing states, nephrotic syndrome, enteropathies, and aging.

Primary B-cell immunodeficiencies may be treated such as selective IgA deficiency, selective IgM deficiency, selective IgG sub class deficiency, X-linked agammaglobulinemia and variable hypogammaglobulinemia.

Finally, peptides according to the present invention may be used to treat surgical, gynecological and ENT purulent diseases.

### EXAMPLE 1

### EFFECT OF IMMUNE SYSTEM OF HEALTHY GUINEA PIGS

Male guinea pigs are used in the following test.
Most of the animals are treated daily with a single dose (i.m.) of the pentapeptide (linear monomer Glx=Glu) of microgram/kg for five days. Control animals are treated with single daily doses of 0.5 ml (i.m.) of normal saline.

Tested parameters: clinical blood examination, non-specific resistance by lysosomal cationic test; "active" T-lymphocytes and total T-lymphocytes (E-RFC), B-lymphocytes (EAC-RFC) are measured in blood, thymus, lymph nodes, spleen and red bone marrow. Blood lymphocyte functional activity is evaluated by leukocyte migration inhibition (LMI) with ConA. Histological examinations of thymus, spleen, lymph nodes, bone marrow and adrenals are carried out. All of these indicia are measured on 10th and 20th days after onset of the treatment.

The peptide stimulates lymphoid cells proliferation and differentiation in thymus and bone marrow; predominant T-lymphocyte stimulation is observed, followed by stimulation of both T- and B-lymphocytes. The peptide causes increased level of mitotic activity in lymph nodes, spleen, and especially bone marrow.

### EXAMPLE 2

### EFFECT ON IMMUNE SYSTEM

Male guinea pigs are used in the following test.

Guinea pigs are exposed to irradiation in a total dose 1Gy.target-skin distance - 70 cm; time of exposure - 2'48" Device: 180 kV; 15 mA; filter 0.5 Cu Treatment: i.m. single daily 1 microg. active ingredient per kg for 5 days

Treatment of controls: normal saline 0.5 ml i.m. single daily for 5 days.

Leukocyte and lymphocyte levels are measured in peripheral blood periodically after irradiation.

The peptide stimulates proliferation of blood lymphoid cells resulted in restoration of leukocyte and lymphocyte levels.

In a second test male guinea pigs are used, and the same regimen is followed.

There are two controls - irradiated and nonirradiated. Parameters are evaluated on the 8th and 21st days after irradiation. Tested parameters: clinical blood examination, non-specific resistance by lysosomal cationic test; "active" T-lymphocytes and total T-lymphocytes (E-RFC), B-lymphocytes (EAC-RFC) were measured in blood, thymus, lymph nodes, spleen and red bone marrow. Blood lymphocyte functional activity is evaluated by leukocyte migration inhibition (LMI) with ConA. Histological examinations of thymus, spleen, lymph nodes, bone marrow and adrenals are carried out.

The peptide use in irradiated animals accelerates T-lymphocyte maturation and their migration to peripheral immune organs in early terms of observation. In the later stage of the study effects are more pronounced in the enhancement of proliferation and differentiation in both central and peripheral organs of the immune system. Administration restors peripheral blood lymphocytes and neutrophil functional activity.

### EXAMPLE 3

### EFFECTS IN THYMECTEMIZED GUINEA PIGS

Model: Thymectomy (removal of thymus): mongrel male guinea pigs

Treatment: i.m. single daily 1 microg active ingredient per kg for 10 days

Treatment of controls: normal saline 0.5 ml i.m. single daily for 10 days (there are two controls - thymectomized and sham-operated).

Parameters are determined on the 15th day after onset of the treatment.

Tested parameters: clinical blood examination, "active" T-lymphocytes and total T-lymphocytes (E-RFC), B-lymphocytes (EAC-RFC) are measured in blood, thymus, lymph nodes, spleen and red bone marrow.

The peptide use in thymectomized animals does not stimulate lymphoid cells differentiation, but, on the contrary, does suppress it to some degree.

### EXAMPLE 4

### EFFECT ON SUPERFICIAL RECEPTORS EXPRESSION ON T- AND B-LYMPHOCYTES

Model: A. This work is designed to study the restoration of superficial receptors on lymphocytes after proteolytic digestion or after severe secondary immunodeficiency. Thymocytes obtained from guinea-pig are trypsinized and then their rosette-forming capacity with rabbit erythrocytes (E-RFC) are evaluated. The cells are incubated with the peptide in concentrations 1, 10 and 100 microg/ml. There are two controls - intact thymocytes (not trypsinized) and trypsinized thymocytes not incubated with the peptide.

The peptide is the most active in concentration 10 microg/ml - its biological activity made up 78.9% (percentage of rosette-forming capacity restoration).

B. B-lymphocytes are obtained from patients with streptococcal and staphylococcal skin diseases and show highly pronounced secondary immunodeficiency. The number of cells carrying Ig-receptors before and after incubation with the peptide are measured (by means of FITC-labelled sera against human Ig).

The peptide in concentration 1 microg/ml causes significant increase of cells carrying Ig-receptors of different types.

### EXAMPLE 5

### ERYTHROPOIETIC EFFECTS

This test is designed to study posthemorrhagic anemia (acute blood loss caused by taking blood from retroorbital sinus), and hemolytic anemia induced by phenylhydrazine hydrochloride (120 mg active ingredients/kg 30 Balb/c-mice and 30 CBA-mice).

The peptide is injected intraperitoneally in doses 100 and 150 microg per kg, 3 hours and 1 day after intervention modelling anemia, for 5 days.

Tested parameters: RBC, leukocytes, reticulocytes, Hb, Hct
1. In posthemorrhagic anemia the most pronounced alterations of tested parameters are observed on several days after the invasion: erythrocytes drop compared to control, reticulocytes rise, leukocytes are also increased. After administration, on the 6th day, RBC count rises up to 7.1 mln/ml; Hb level and plasma/formed elements ration restoration are more rapid. The peptide is effective in doses of 150 microg/kg.
2. In hemolytic phenylhydrazine-induced anemia the most pronounced hemodepression arises several days after beginning administration. RBC drops, Hb is diminished, reticulocytes increase. After several days of administration, erythrocytes increase and remain level. Thus, the peptide has erythropoietic effect in anemias of different genesis.

### EXAMPLE 6

### INFLUENCE ON COLONY-FORMING ACTIVITY

This test is designed to study macrophage precursors. Cultured cells used are guinea-pig myelokaryocytes. The peptide is added to cell culture in concentrations 1.0, 0.001, 0.00001 and 0.0000001 microg/ml.

The peptide stimulates macrophage precursors colony-forming activity in concentration starting from 0.0000001 microg/ml.

### EXAMPLE 7

### HEMOSTIMULATING EFFECT

The test is designed to study hemodepression induced by 5-fluorouracil injected i.p. in a dose 175 mg per kg (172 male CBA-mice). Treatment: the peptide is administered i.p. starting from 4th day after 5-FU injection in doses 0.00001, 0.001, 0.01, 0.1, 1.0 mg/kg for 5 days.

Treatment of controls: normal saline i.p. for 5 days.

Tested parameters: peripheral blood count and bone marrow differential count.

The peptides promote active restoration of hemopoiesis. This results in normalization of leukocytes and all CBC parameters. In bone marrow the peptide causes restoration of cellularity normalization of all lines of hemopoiesis. The peptide is active starting from 0.001 mg/kg.

## Claims

1. A substantially pure compound of at least five amino acids of the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

2. The compound of claim 1 wherein the compound is Thr-Pro-Glx-Glx-Lys or a pharmaceutically acceptable salt thereof.

3. The compound of claim 1 wherein the compound is Thr-Pro-Glu-Glu-Lys or a pharmaceutically acceptable salt thereof.

4. The compound of claim 1 wherein the compound is Thr-Pro-Gln-Gln-Lys or a pharmaceutically acceptable salt thereof.

5. The compound of claim 1 wherein R" is -H.

6. The compound of claim 1 wherein R' is Thr-Pro-.

7. The compound of claim 1 wherein the compound is Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val or pharmaceutically acceptable salts thereof.

8. The compound of any of claims 1-7 which is a pharmaceutically acceptable salt selected from sodium, potassium, ammonium, guanidinium and zinc.

9. A pharmaceutical composition comprising a pharmaceutically acceptable vehicle and an effective amount of a substantially pure compound of the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and-Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

10. The pharmaceutical composition of claim 9 wherein the compound is a pharmaceutically acceptable salt selected from sodium, potassium, ammonium, guanidinium and zinc.

11. The pharmaceutical composition of claim 9 wherein the compound is Thr-Pro-Glx-Glx-Lys or a pharmaceutically acceptable salt thereof.

12. The pharmaceutical composition of claim 9 wherein the compound is Thr-Pro-Glu-Glu-Lys or a pharmaceutically acceptable salt thereof.

13. The pharmaceutical composition of claim 9 wherein the compound is Thr-Pro-Gln-Gln-Lys or a pharmaceutically acceptable salt thereof.

14. The pharmaceutical composition of claim 9 wherein the compound is Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val or pharmaceutically acceptable salts thereof.

15. The pharmaceutical composition of claim 11 for the treatment of an immunodepressed or immunodeficient condition.

16. The pharmaceutical composition of claim 11 for the treatment of an elevated immune state.

17. The pharmaceutical composition of claim 11 for the treatment of an opportunistic infection associated with an immunodepressed or immunodeficient state.

18. The pharmaceutical composition of claim 15 wherein the immunodepressed or immunodeficient condition is AIDS.

19. The pharmaceutical composition of claim 15 wherein the immunodepressed or immunodeficient condition is a lymphopenia or neutropenia.

20. The pharmaceutical composition of any of claims 9-19 formulated as an intramuscular injection solution comprising 0.001% to 0.01% of the compound by weight.

21. The pharmaceutical composition of any of claims 9-19 formulated as an intramuscular injection solution wherein the pharmaceutically acceptable vehicle is an aqueous solution.

22. The pharmaceutical composition of any of claims 9-19 wherein the pharmaceutically acceptable vehicle is suitable for parenteral administration.

23. The pharmaceutical composition of any of claims 9-19 in the form of a tablet, capsule or suppository.

24. The pharmaceutical composition of any of claims 9-19 comprising a dose of the compound of 10 µg to 50 µg formulated for intramuscular administration.

25. The pharmaceutical composition of any of claims 9-19 comprising a dose of the compound of 50 µg to 100 µg formulated for intramuscular administration.

26. Use of a substantially pure compound for the manufacture of a pharmaceutical composition for the treatment of an immunodeficient, immunodepressed or elevated immune state wherein the compound has the formula R'-Glx-Glx-Lys-R" or a pharmaceutically acceptable salt thereof, wherein:
R' is a first amino acid sequence selected from Thr-Pro-, Thr-Ala-, Ser-Ala-, Ser-Pro-, Ser-Ser- and Leu-Thr-Ala-;
Glx is Glu or Gln;
R" is -H or a second amino acid sequence selected from -Ala, -Ala-Ala or -Ala-Val;
wherein all amino acids are in the L- configuration, with the proviso that R'-Glx-Glx-Lys-R" is not Thr-Ala-Glx-Glx-Lys.

27. The use of claim 26 wherein the compound is a pharmaceutically acceptable salt selected from sodium, potassium, ammonium, guanidinium or zinc.

28. The use of claim 26 wherein the compound is Thr-Pro-Glx-Glx-Lys or a pharmaceutically acceptable salt thereof.

29. The use of claim 26 wherein the compound is Thr-Pro-Glu-Glu-Lys or a pharmaceutically acceptable salt thereof.

30. The use of claim 26 wherein the compound is Thr-Pro-Gln-Gln-Lys or a pharmaceutically acceptable salt thereof.

31. The use of claim 26 wherein the compound is Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val or pharmaceutically acceptable salts thereof.

32. The use of any of claims 26 to 31 for the treatment of an immunodepressed or immunodeficient immune state.

33. The use of claim 32 wherein the immunodepressed or immunodeficient immune state is AIDS.

34. The use of claim 32 wherein the immunodepressed or immunodeficient immune state is a lymphopenia or neutropenia.

35. The use of any of claims 26 to 31 for the treatment of a elevated immune state.

36. The use of any of claims 26 to 31 for the treatment of an opportunistic infection associated with an immunodepressed or immunodeficient immune state.

## Patentansprüche

1. Im wesentlichen reine Verbindung aus mindestens fünf Aminosäuren der Formel R'-Glx-Glx-Lys-R" oder ein pharmazeutisch akzeptables Salz hiervon, worin:
R' eine erste Aminosäuresequenz ist, die aus Thr-Pro-, Thr-Ala-, Ser-Ala-, Ser-Pro-, Ser-Ser- und Leu-Thr-Ala - ausgewählt ist;
Glx Glu oder Gln ist;
R" -H oder eine zweite Aminosäuresequenz ist, die aus - Ala, -Ala-Ala oder -Ala-Val ausgewählt ist;
wobei alle Aminosäuren in der L-Konfiguration vorliegen, mit der Maßgabe, dass R'-Glx'-Glx-Lys-R" nicht Thr-Ala-Glx-Glx-Lys ist.

2. Verbindung nach Anspruch 1, wobei die Verbindung Thr-Pro-Glx-Glx-Lys oder ein pharmazeutisch akzeptables Salz davon ist.

3. Verbindung nach Anspruch 1, wobei die Verbindung Thr-Pro-Glu-Glu-Lys oder ein pharmazeutisch akzeptables Salz davon ist.

4. Verbindung nach Anspruch 1, wobei die Verbindung Thr-Pro-Gln-Gln-Lys oder ein pharmazeutisch akzeptables Salz davon ist.

5. Verbindung nach Anspruch 1, wobei R" -H ist.

6. Verbindung nach Anspruch 1, wobei R' Thr-Pro- ist.

7. Verbindung nach Anspruch 1, wobei die Verbindung Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ist, oder pharmazeutisch akzeptable Salze hiervon.

8. Verbindung nach einem der Ansprüche 1 bis 7, welche ein pharmazeutisch akzeptables Salz ist, das aus Natrium, Kalium, Ammonium, Guanidinium und Zink ausgewählt ist.

9. Pharmazeutische Zusammensetzung, umfassend ein pharmazeutisch akzeptables Vehikel und eine wirksame Menge einer im wesentlichen reinen Verbindung der Formel R'-Glx-Glx-Lys-R" oder eines pharmazeutisch akzeptablen Salzes hiervon, worin:
R' eine erste Aminosäuresequenz ist, die aus Thr-Pro-, Thr-Ala-, Ser-Ala-, Ser-Pro-, Ser-Ser- und Leu-Thr-Ala - ausgewählt ist,
Glx Glu oder Gln ist;
R" -H oder eine zweite Aminosäuresequenz ist, die aus - Ala, -Ala-Ala oder -Ala-Val ausgewählt ist;
wobei alle Aminosäuren in der L-Konfiguration vorliegen, mit der Maßgabe, dass R'-Glx-Glx-Lys-R" nicht Thr-Ala-Glx-Glx-Lys ist.

10. Pharmazeutische Zusammensetzung nach Anspruch 9, worin die Verbindung ein pharmazeutisch akzeptables Salz ist, das aus Natrium, Kalium, Ammonium, Guanidinium und Zink ausgewählt ist.

11. Pharmazeutische Zusammensetzung nach Anspruch 9, worin die Verbindung Thr-Pro-Glx-Glx-Lys oder ein pharmazeutisch akzeptables Salz davon ist.

12. Pharmazeutische Zusammensetzung nach Anspruch 9, worin die Verbindung Thr-Pro-Glu-Glu-Lys oder ein pharmazeutisch akzeptables Salz davon ist.

13. Pharmazeutische Zusammensetzung nach Anspruch 9, worin die Verbindung Thr-Pro-Gln-Gln-Lys oder ein pharmazeutisch akzeptables Salz davon ist.

14. Pharmazeutische Zusammensetzung nach Anspruch 9, worin die Verbindung Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ist, oder pharmazeutisch akzeptable Salze hiervon.

15. Pharmazeutische Zusammensetzung nach Anspruch 11, zur Behandlung eines immunodepressiven oder immunodefizienten Zustands.

16. Pharmazeutische Zusammensetzung nach Anspruch 11, zur Behandlung eines erhöhten Immunzustands.

17. Pharmazeutische Zusammensetzung nach Anspruch 11, zur Behandlung einer opportunistischen Infektion, die mit einem immunodepressiven oder immunodefizienten Zustand in Zusammenhang steht.

18. Pharmazeutische Zusammensetzung nach Anspruch 15, wobei der immunodepressive oder immunodefiziente Zustand AIDS ist.

19. Pharmazeutische Zusammensetzung nach Anspruch 15, wobei der immunodepressive oder immunodefiziente Zustand eine Lymphverminderung oder Neutropenie ist.

20. Pharmazeutische Zusammensetzung nach einem der Ansprüche 9-19, die als intramuskuläre Injektionslösung formuliert ist, welche 0,001 Gew.-% bis 0,01 Gew.-% der Verbindung umfasst.

21. Pharmazeutische Zusammensetzung nach einem der Ansprüche 9-19, die als eine intramuskuläre Injektionslösung formuliert ist, wobei das pharmazeutisch akzeptable Vehikel eine wässerige Lösung ist.

22. Pharmazeutische Zusammensetzung nach einem der Ansprüche 9-19, wobei das pharmazeutisch akzeptable Vehikel zur parenteralen Verabreichung geeignet ist.

23. Pharmazeutische Zusammensetzung nach einem der Ansprüche 9-19 in Form einer Tablette, Kapsel oder eines Zäpfchens.

24. Pharmazeutische Zusammensetzung nach einem der Ansprüche 9-19, welche eine Dosierung der Verbindung von 10 µg bis 50 µg umfasst und zur intramuskulären Verabreichung formuliert ist.

25. Pharmazeutische Zusammensetzung nach einem der Ansprüche 9-19, welche eine Dosierung der Verbindung von 50 µg bis 100 µg umfasst und zur intramuskulären Verabreichung formuliert ist.

26. Verwendung einer im wesentlichen reinen Verbindung zur Herstellung einer pharmazeutischen Zusammensetzung zur Behandlung eines immunodefizienten, immunodepressiven oder eines erhöhten Immunzustands, wobei die Verbindung die Formel R'-Glx-Glx-Lys-R" aufweist, oder ein pharmazeutisch akzeptables Salz hiervon, worin:
R' eine erste Aminosäuresequenz ist, die aus Thr-Pro-, Thr-Ala-, Ser-Ala-, Ser-Pro-, Ser-Ser- und Leu-Thr-Ala - ausgewählt ist;
Glx Glu oder Gln ist;
R" -H oder eine zweite Aminosäuresequenz ist, die aus - Ala, -Ala-Ala oder -Ala-Val ausgewählt ist;
wobei alle Aminosäuren in der L-Konfiguration vorliegen, mit der Maßgabe, dass R'-Glx-Glx-Lys-R" nicht Thr-Ala-Glx-Glx-Lys ist.

27. Verwendung nach Anspruch 26, wobei die Verbindung ein pharmazeutisch akzeptables Salz ist, das aus Natrium, Kalium, Ammonium, Guanidinium oder Zink ausgewählt ist.

28. Verwendung nach Anspruch 26, wobei die Verbindung Thr-Pro-Glx-Glx-Lys oder ein pharmazeutisch akzeptables Salz davon ist.

29. Verwendung nach Anspruch 26, wobei die Verbindung Thr-Pro-Glu-Glu-Lys oder ein pharmazeutisch akzeptables Salz davon ist.

30. Verwendung nach Anspruch 26, wobei die Verbindung Thr-Pro-Gln-Gln-Lys oder ein pharmazeutisch akzeptables Salz davon ist.

31. Verwendung nach Anspruch 26, wobei die Verbindung Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ist, oder pharmazeutisch akzeptable Salze hiervon.

32. Verwendung nach einem der Ansprüche 26 bis 31, zur Behandlung eines immunodepressiven oder immunodefizienten Immunzustands.

33. Verwendung nach Anspruch 32, wobei der immunodepressive oder immunodefiziente Zustand AIDS ist.

34. Verwendung nach Anspruch 32, wobei der immunodepressive oder immunodefiziente Zustand eine Lymphverminderung oder Neutropenie ist.

35. Verwendung nach einem der Ansprüche 26 bis 31, zur Behandlung eines erhöhten Immunzustands.

36. Verwendung nach einem der Ansprüche 26 bis 31, zur Behandlung einer opportunistischen Infektion, die mit einem immunodepressiven oder immunodefizienten Immunzustand in Zusammenhang steht.

## Revendications

1. Composé substantiellement pur, d'au moins cinq amino-acides, de formule R'-Glx-Glx-Lys-R" ou un de ses sels pharmaceutiquement acceptables, formule dans laquelle :
R' représente une première séquence d'amino-acides choisie entre Thr-Pro-, Thr-Ala-, Ser-Ala-, Ser-Pro-, Ser-Ser- et Leu-Thr-Ala- ;
Glx représente Glu ou Gln ;
R" représente -H ou une seconde séquence d'amino-acides choisie entre -Ala, -Ala-Ala et -Ala-Val ;
tous les amino-acides étant en configuration L, sous réserve que R'-Glx-Glx-Lys-R" ne représente pas Thr-Ala-Glx-Glx-Lys.

2. Composé suivant la revendication 1, qui consiste en Thr-Pro-Glx-Glx-Lys ou un de ses sels pharmaceutiquement acceptables.

3. Composé suivant la revendication 1, qui consiste en Thr-Pro-Glu-Glu-Lys ou un de ses sels pharmaceutiquement acceptables.

4. Composé suivant la revendication 1, qui consiste en Thr-Pro-Gln-Gln-Lys ou un de ses sels pharmaceutiquement acceptables.

5. Composé suivant la revendication 1, dans lequel R" représente -H.

6. Composé suivant la revendication 1, dans lequel R' représente Thr-Pro-.

7. Composé suivant la revendication 1, qui consiste en Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ou leurs sels pharmaceutiquement acceptables.

8. Composé suivant l'une quelconque des revendications 1 à 7, qui est un sel pharmaceutiquement acceptable choisi entre les sels de sodium, de potassium, d'ammonium, de guanidinium et de zinc.

9. Composition pharmaceutique comprenant un véhicule pharmaceutiquement acceptable et une quantité efficace d'un composé substantiellement pur de formule R'-Glx-Glx-Lys-R" ou d'un de ses sels pharmaceutiquement acceptables, formule dans laquelle :
R' représente une première séquence d'amino-acides choisie entre Thr-Pro-, Thr-Ala-, Ser-Ala-, Ser-Pro-, Ser-Ser- et Leu-Thr-Ala- ;
Glx représente Glu ou Gln ;
R" représente -H ou une seconde séquence d'amino-acides choisie entre -Ala, -Ala-Ala et -Ala-Val ;
tous les amino-acides étant en configuration L, sous réserve que R'-Glx-Glx-Lys-R" ne représente pas Thr-Ala-Glx-Glx-Lys.

10. Composition pharmaceutique suivant la revendication 9, dans laquelle le composé est un sel pharmaceutiquement acceptable choisi entre les sels de sodium, de potassium, d'ammonium, de guanidinium et de zinc.

11. Composition pharmaceutique suivant la revendication 9, dans laquelle le composé consiste en Thr-Pro-Glx-Glx-Lys ou un de ses sels pharmaceutiquement acceptables.

12. Composition pharmaceutique suivant la revendication 9, dans laquelle le composé consiste en Thr-Pro-Glu-Glu-Lys ou un de ses sels pharmaceutiquement acceptables.

13. Composition pharmaceutique suivant la revendication 9, dans laquelle le composé consiste en Thr-Pro-Gln-Gln-Lys ou un de ses sels pharmaceutiquement acceptables.

14. Composition pharmaceutique suivant la revendication 9, dans laquelle le composé consiste en Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ou leurs sels pharmaceutiquement acceptables.

15. Composition pharmaceutique suivant la revendication 11, pour le traitement d'un état immunodéprimé ou immunodéficient.

16. Composition pharmaceutique suivant la revendication 11, pour le traitement d'un état d'immunité élevé.

17. Composition pharmaceutique suivant la revendication 11, pour le traitement d'une infection opportuniste associée à un état immunodéprimé ou immunodéficient.

18. Composition pharmaceutique suivant la revendication 15, dans laquelle l'état immunodéprimé ou immunodéficient est le SIDA.

19. Composition pharmaceutique suivant la revendication 15, dans laquelle l'état immunodéprimé ou immunodéficient est une lymphopénie ou une neutropénie.

20. Composition pharmaceutique suivant l'une quelconque des revendications 9 à 19, formulée à l'état de solution injectable intramusculaire comprenant 0,001% à 0,01% du composé, en poids.

21. Composition pharmaceutique suivant l'une quelconque des revendications 9 à 19, formulée à l'état de solution injectable intramusculaire, dans laquelle le véhicule pharmaceutiquement acceptable est une solution aqueuse.

22. Composition pharmaceutique suivant l'une quelconque des revendications 9 à 19, dans laquelle le véhicule pharmaceutiquement acceptable est apte à l'administration parentérale.

23. Composition pharmaceutique suivant l'une quelconque des revendications 9 à 19, sous forme d'un comprimé, d'une capsule ou d'un suppositoire.

24. Composition pharmaceutique suivant l'une quelconque des revendications 9 à 19, comprenant une dose du composé de 10 µg à 50 µg formulée pour l'administration intramusculaire.

25. Composition pharmaceutique suivant l'une quelconque des revendications 9 à 19, comprenant une dose du composé de 50 µg à 100 µg formulée pour l'administration intramusculaire.

26. Utilisation d'un composé substantiellement pur pour la production d'une composition pharmaceutique destinée au traitement d'un état immunodéficient, d'un état immunodéprimé ou d'un état d'immunité élevé, dans laquelle le composé répond à la formule R'-Glx-Glx-Lys-R" ou un de ses sels pharmaceutiquement acceptables, formule dans laquelle :
R' représente une première séquence d'amino-acides choisie entre Thr-Pro-, Thr-Ala-, Ser-Ala-, Ser-Pro-, Ser-Ser- et Leu-Thr-Ala- ;
Glx représente Glu ou Gln ;
R" représente -H ou une seconde séquence d'amino-acides choisie entre -Ala, -Ala-Ala et -Ala-Val ;
tous les amino-acides étant en configuration L, sous réserve que R'-Glx-Glx-Lys-R" ne représente pas Thr-Ala-Glx-Glx-Lys.

27. Utilisation suivant la revendication 26, dans laquelle le composé est un sel pharmaceutiquement acceptable choisi entre les sels de sodium, de potassium, d'ammonium, de guanidinium et de zinc.

28. Utilisation suivant la revendication 26, dans laquelle le composé consiste en Thr-Pro-Glx-Glx-Lys ou un de ses sels pharmaceutiquement acceptables.

29. Utilisation suivant la revendication 26, dans laquelle le composé consiste en Thr-Pro-Glu-Glu-Lys ou un de ses sels pharmaceutiquement acceptables.

30. Utilisation suivant la revendication 26, dans laquelle le composé consiste en Thr-Pro-Gln-Gln-Lys ou un de ses sels pharmaceutiquement acceptables.

31. Utilisation suivant la revendication 26, dans laquelle le composé consiste en Leu-Thr-Ala-Glx-Glx-Lys-Ala, Leu-Thr-Ala-Glx-Glx-Lys-Ala-Ala, Leu-Thr-Ala-Glx-Glx-Lys-Ala-Val ou leurs sels pharmaceutiquement acceptables.

32. Utilisation suivant l'une quelconque des revendications 26 à 31, pour le traitement d'un état immunitaire immunodéprimé ou immunodéficient.

33. Utilisation suivant la revendication 32, dans laquelle l'état immunitaire immunodéprimé ou immunodéficient est le SIDA.

34. Utilisation suivant la revendication 32, dans laquelle l'état immunitaire immunodéprimé ou immunodéficient est une lymphopénie ou une neutropénie.

35. Utilisation suivant l'une quelconque des revendications 26 à 31 pour le traitement d'un état d'immunité élevé.

36. Utilisation suivant l'une quelconque des revendications 26 à 31 pour le traitement d'une infection opportuniste associée à un état immunitaire immunodéprimé ou immunodéficient.
